# EUROPEAN PATENT SPECIFICATION

(11) **EP 3 448 215 B1**
(45) Date of publication and mention of the grant of the patent: **04.11.2020**
(21) Application number: 17722398.9
(22) Date of filing: 25.04.2017
(51) Int. Cl.: A47K 5/12, A47K 10/32, G08B 21/24

(54) **REWARD HYGIENE EQUIPMENT**
HYGIENEAUSRÜSTUNG MIT BELOHNUNG
ÉQUIPEMENT D'HYGIÈNE À RÉCOMPENSE

(30) Priority: 25.04.2016 WO PCT/EP2016/059147
(43) Date of publication of application: 06.03.2019
(73) Proprietor: Essity Hygiene and Health Aktiebolag, 405 03 Göteborg (SE)
(72) Inventor: BERLAND, Carolyn, 405 03 Göteborg (SE)
(74) Representative: Hoffmann Eitle
(86) International application number: PCT/EP2017/059743
(87) International publication number: WO 2017/186688

(56) References cited:
- EP-A1- 2 805 657
- US-A1- 2011 169 645
- US-A1- 2015 062 073
- US-A1- 2015 134 357
- US-B1- 7 782 214

## Description

### Technical field

The present disclosure generally relates to hygiene equipment, such as soap, disinfectant, and/or towel dispensers, and the like. More particularly, the present disclosure relates to such hygiene equipment for promoting the use thereof and, in turn, for improving and/or maintaining a compliance rate with respect to a target usage of hygiene equipment. The present disclosure likewise relates to a corresponding method for operating hygiene equipment.

### Background

Hygiene equipment is commonplace today in many facilities, such as hospitals, medical service centers, intensive care units, day clinics, private practices, lavatories, rest rooms, hotels, manufacturing sites, administration and office buildings, and, in general, places and facilities that are accessible to the public or to a considerable number of individuals. The mentioned hygiene equipment thereby includes various types of individual devices and installations such as soap dispensers, dispensers for disinfectant solutions, gels or substances, towel dispensers, glove dispensers, tissue dispensers, hand dryers, sinks, radiation assisted disinfectant points, and the like.

Although such hygiene equipment is commonplace today in many places, the use thereof by the individuals visiting these places or working in these places is still oftentimes not satisfactory. For example, hospitals, and, in general, medical service centers often suffer from hygiene deficiencies, which, in turn, may lead to the spread of infections and related diseases. In particular, such insufficient hygiene amongst medical care personnel coming into close contact with patients and bodily fluids can lead to the spread of infectious diseases amongst the personnel and other patients. It is also known that infections by highly resistant bacteria pose a severe problem in such places, above all, hospitals.

At the same time, however, it is known that hygiene, and, in particular, hand hygiene, is an important factor as far as the spread of infectious diseases are concerned. Specifically, medical care personnel should make proper use of hand hygiene as often as possible so that the spread of bacteria and other disease causing substances is minimized. The usage of such hygiene equipment, however, is dependent on - amongst others - the cooperation and will shown by the individuals working in these places or visiting such places. In other words, an important factor remains the fact that individuals may not make use of installed and provided hygiene equipment although they are supposed to. Furthermore, it is generally accepted that an increased use of hygiene equipment can substantially contribute in reducing the spread of bacteria and the like, which, in turn, can drastically reduce the appearance of related infections and diseases.

Although there are many compliance monitoring schemes known in the arts, there is still a need for improved hygiene equipment that specifically can promote the actual usage thereof. As a consequence, such improved hygiene equipment may be able to contribute in reducing the spread of infectious diseases in general and in particular also in the context of the above mentioned facilities, such as hospitals.

US 2011/0169645 A1 discloses as a so-called random event the use of a dispenser. Once detected inside an award period, the random event of using the dispenser triggers the award. The remaining time of that interval needs then first to elapse before the next random event can trigger an award. This requires that an additional entity takes responsibility for setting award period each time an award has occurred. Further, the respective teaching does not contemplate a fully independent detection of a usage, the local evaluation of a random element and the corresponding initiation of a reward action.

US 7,782,214 B1 discloses a hygiene device that also dispenses information in response to be used and that this device may work like a lottery. Larger prizes can be won if subject to a central management. However, the disclosure does not provide a teaching on how to effect a reward action locally.

### Summary

The mentioned problems and drawbacks are addressed by the subject matter of the independent claims. Further preferred embodiments are defined in the dependent claims.

### Brief description of the drawings

Embodiments of the present invention, which are presented for better understanding the inventive concepts but which are not to be seen as limiting the invention, will now be described with reference to the figures in which:
- Figure 1A: shows a schematic view of a piece of hygiene equipment according to an embodiment of the invention;
- Figure 1B and 1C: show schematic views of possible implementations of a sensor section of a piece of hygiene equipment according to an embodiment of the invention;
- Figures 2A to 2E: show schematic views of further pieces of hygiene equipment according to corresponding further embodiments of the invention;
- Figure 2FE: shows a schematic view of a piece of hygiene equipment according to a further embodiment of the invention that does not rely on electronics;
and
- Figure 3: shows a flowchart of a general method embodiment of the present invention.

### Detailed description

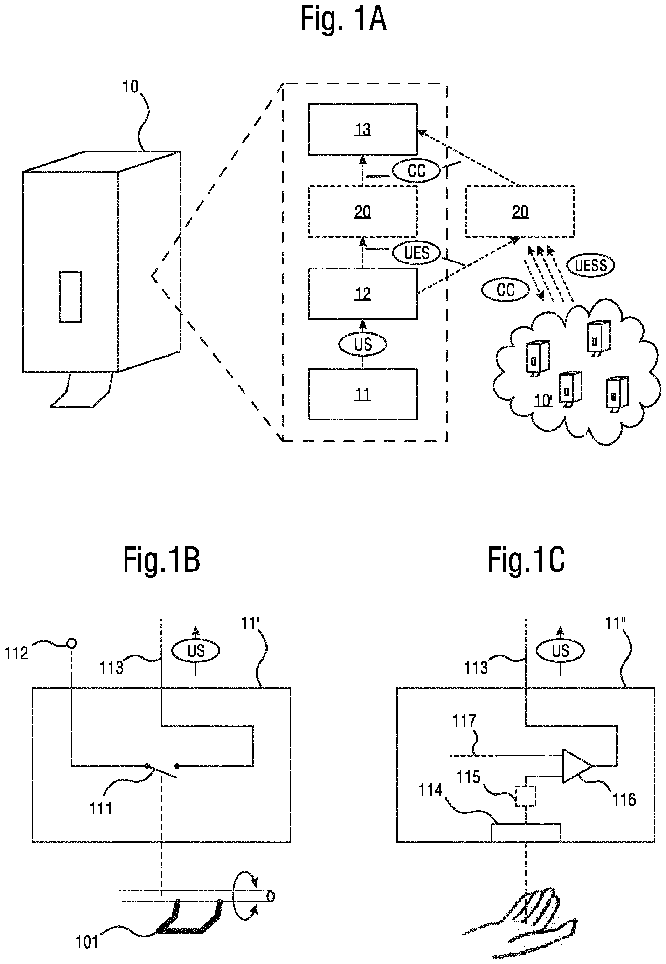

Figure 1A shows a schematic view of an exemplary piece of hygiene equipment according to an embodiment of the present invention. Specifically, it is shown a dispenser 10 that may be installed in a facility for dispensing soap or a disinfectant liquid. In general, however, the shown piece of hygiene equipment 10 is only an example within the present embodiment and can be well replaced by any suitable piece of hygiene equipment, including also towel dispensers, hand wash installations, sinks, hand dryers, radiation assisted disinfection and hygiene equipment, and the like. Therefore, the piece of hygiene equipment as subject to the present disclosure may be generally defined as any equipment that is able to perform a hygienic action on an individual, a corresponding body part, and/or equipment or tools used and employed by such an individual. As a consequence, it is assumed in the context of the present disclosure that the use of hygiene equipment can contribute avoiding the spread of infectious or elsehow hazardous bacteria, viruses, and or substances.

The shown piece of hygiene equipment 10 comprises in the present invention a sensor section 11 that is configured to generate a usage signal (US) in response to an operator using the piece of hygiene equipment. For example, an operator operates the equipment 10 for dispensing an amount of soap for subsequently washing his/her hands or an amount of disinfectant solution or substance for disinfecting the hands or other parts of his/her body. In response of such a usage, the sensor section 11 generates the usage signal (US) upon, for example, detection of an action (e.g. movement of a lever), or an ejection of the substance or material to be dispensed. In this context, a sensor may comprise any one of a switch, a magnetic sensor, a Hall element, a light barrier, an infrared light source and/or sensor, a proximity sensor, and the like. Thus, the sensor section 11 in such embodiments generates the usage signal (US) in the form of an electric signal that can be applied to further interacting sections of the piece of hygiene equipment.

Figure 1B shows a schematic view of a possible implementation of a sensor section 11' of a piece of hygiene equipment according to, for example, the embodiment as described above in conjunction with Figure 1A. In such an implementation, for example, a lever 101 to be actuated by a user to expel soap or a disinfectant substance can mechanically interact with an electric switch 111 that closes or opens an electrical connection to a voltage or current source 112. The change of a voltage or current level can then be detected by any interacting section by way of an electrical connection 113 (wire, circuit board path, etc.).

In another implementation, as shown in Figure 1C, an electric circuit may be provided to evaluate a primary electrical signal from a primary sensor of a corresponding sensor section 11". A primary sensor 114 may be a Hall element, light sensor, infrared sensor, capacitive sensor, or the like that is able to change its respective electric characteristics in response to a situation which corresponds to a usage of the piece of hygiene equipment. For example, the sensor 114 may be in the form of a (infrared) light or capacitive sensor that changes its characteristics when a hand of an operator is in the vicinity of the piece of hygiene equipment during ejection of soap, disinfectant, or during any other suitable hygiene action. In order to generate an again usage signal (US) in the form of an electrical signal 113, the primary electrical signal from the sensor 114, may be, optionally, averaged over time, filtered, smoothened, and/or amplified in a signal conditioning circuit 115 (e.g. filter circuit of RC- or related type, amplifier, etc.). This signal can then be supplied to a comparator circuit 116 the output of which can then be used as the usage signal (US). In the case of an operational amplifier a reference signal 117 may be employed, whereas in other circuits, e.g. Schmitt trigger, no such reference may be needed.

With again reference to Figure 1A, the piece of hygiene equipment 10 further comprises a detection section 12 that is configured to forward, in response to receiving the usage signal US, a usage event signal UES to a determination section 20. In some embodiments, said determination section 20 forms part of the piece of hygiene equipment 10 itself, whereas in other embodiments of the present invention, the signal UES is forwarded to a determination section 20 that is located remotely from the piece of equipment 10. In the embodiments where said determination section 20 forms part of the piece of hygiene equipment 10 itself, the detection section 12 may be implemented as a mere connection from the sensor section 11 to the determination section 20 and the usage signal (US) may be the same electrical signal as the usage event signal (UES). In other words, in such embodiments, the determination section 20 is configured to receive the electrical signal directly from the sensor section 11 for determining that a related usage event took place.

In the latter embodiments, however, the determination section 20 does not form part of the piece of hygiene equipment 10 itself and consequently is external to or remote from the piece of hygiene equipment 10. In these embodiments, it may be required for the detection section 12 to generate a suitable usage event signal (UES) that is as such different from the usage signal (US) but, naturally, carries a corresponding information content related to whether a usage event has taken place. For such purposes, the detection section 12 may comprise a communication section that is configured to forward a corresponding usage event signal (UES) towards a determination section 20 that forms part of, for example, a server or network entity connected to e.g. the Internet.

Such a communication section may comprise circuits for conducting any wireless or wire bound communication, including, a local area network (LAN) connection, a wireless local area network (WLAN, WiMAX, WiFi) connection, a GSM/GPRS connection, a 3G/4G/LTE connection, a Bluetooth (TM) connection, and the like. In other words, in such embodiments the usage event signal (UES) is generated and forwarded via some kind of network to an entity that implements the determination section 20. As a consequence, the usage event signal (UES) in such embodiments may take the form of one or more protocol packets or messages, whereas the usage signal (US) can be embodied by a simpler electrical signal. The functionalities for correspondingly generating such packets and/or messages on the basis of the usage signal (US) are implanted in these embodiments by the detection section 12.

At this point it should be noted that such a "central" determination section 20 may be provided for a plurality of pieces of hygiene equipment, so that the determination section 20 can be configured to receive UES signals from many dispensers and equipment in the field for determining respective control commands (CC). This is shown in Figure 1A as the optional plurality 10' of further pieces of hygiene equipment, e.g. dispensers of a ward of a hospital etc. These further pieces of hygiene equipment may likewise transmit usage event signals (UESS) to the determination section 20 that can further apply additional rules for providing also the control command (CC) to one of these pieces of hygiene equipment. In this way, for example a specific group or ward may be especially "promoted" by increasing the possibilities that the random rule issues a reward action to the corresponding pieces of hygiene equipment.

The determination section 20 in any way is configured to determine said control command (CC) based on a random rule and the received usage event signal (UES). In particular, the determination section 20 may in response to receiving a usage event signal (UES) employ a randomizer function for determining whether or not a control command (CC) should be forwarded to a control section 13 of the piece of hygiene equipment 10. It is noted that the random rule applied by the detection section 20 may be in general non-predetermined in the sense that it is generally not known, or at least difficult to predict in advance to the operators of the piece of hygiene equipment whether or not a control command is issued based on a received (UES) signal.

This can be implemented by a randomizer circuit which can be implemented as specific code being executed by a hardware processing circuit. Usually, such implementations instruct a processing circuit to read a clock signal or time information, to obtain a reading from an analogue-to-digital converter (ADC), or to access a pre-stored but random decision pattern. As the output of related clock or ADC circuits usually varies with time, any as such known algorithm can be implemented that produces a quasi randomized output from such varying input. For example, a function of time which considerably varies with the time input or a comparison pattern to an inherently fluctuating ADC output may be used. In the case of a decision pattern, a relatively large number of usage events maps to a relatively small number of reward events where, for example, a counter of usage events is employed and a considerable number of counter values with a varying distance from each other correspond to a reward event (i.e. control command issued). In general, therefore, the result of the random rule a priori remains in a closed realm so that it becomes non-predeterminable, or at least difficult to predict for the external environment.

In the embodiments of a remote determination section 20, the control command can be issued based on further additional rules. For example, the origin, time-of-day, etc. of a received UES signal can be taken into account in order to influence the issuance of the control command (CC). In this way, for example, a specific group of hygiene equipment can be promoted by increasing the possibility that a command CC is issued. This can be implemented, for example, by adjusting a fraction of randomizer results that yield the control command (CC). In this way not only a specific group of dispensers can be promoted but also individuals working at these specific places and/or working at specific times. As far as the latter times are concerned, one embodiment envisages to increase the chances for the control command to be issued for a particular time span, so that, for example, a day or night shift of care personnel can be rewarded more often than another (e.g. there can be an increased chance of reward for the night shift if the corresponding individuals were found not using the equipment sufficiently often).

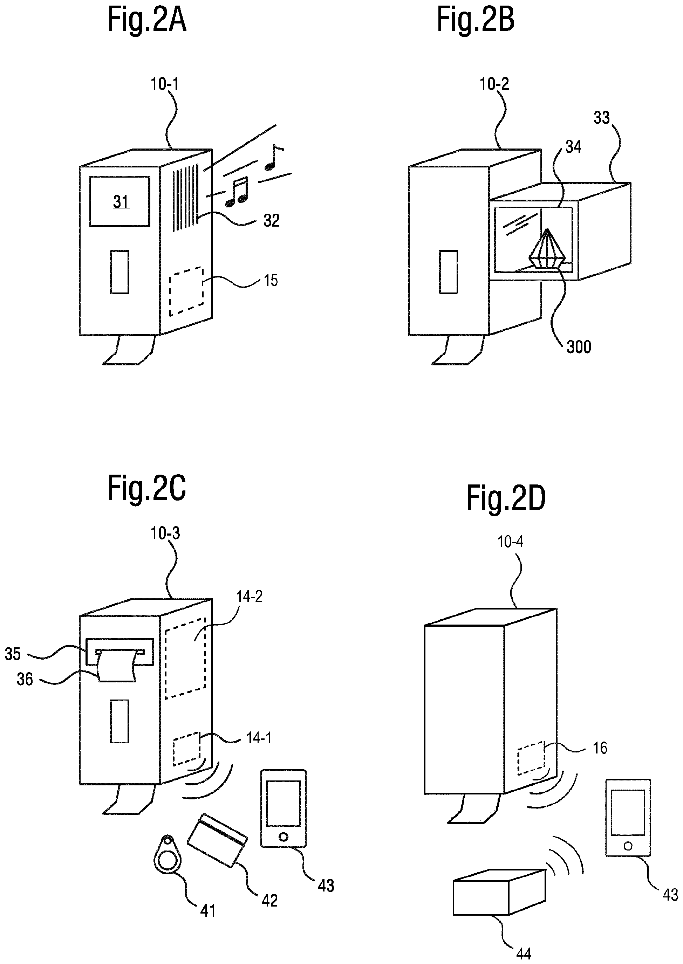

In any way, the already mentioned control section 13 is configured to control a reward action in response to receiving the control command (CC) from the determination section 20. In general, said reward action is to be understood as any action or operation that is able to reward an operator or user of the hygiene equipment as a specific response to using the equipment. In this way, embodiments of the present invention may generally allow for encouraging the use of hygiene equipment for users. Said reward action may thus be identified as an action which is, at least in part, locally executed by the piece of hygiene equipment so that it can be perceived in the vicinity of the piece of hygiene equipment, and, in particular by the one or more nearby operators that use the equipment. More specific examples for such reward actions are given in the context of the embodiments described in conjunction with Figures 2A to 2E.

Figures 2A to 2E now show schematic views of further pieces of hygiene equipment according to corresponding further embodiments of the invention. Specifically, these figures show further exemplary pieces of hygiene equipment 10-1, 10-2, 10-3, and 10-4 according to further corresponding embodiments of the present invention.

As shown in Figure 2A, a piece of hygiene equipment 10-1 comprises a display 31 as well as an audio output 32. At the same time, the piece of hygiene equipment 10-1 comprises a corresponding section for generating a display output to be output on display 31 and may further comprise a sound output to be output by the audio output 32. As far as the latter sound is concerned, possible examples include acoustic signals, sound jingles, songs, music, speech, and the like and any combination of that, and the reward action will comprise controlling the output of corresponding sound signals. The audio output 32 comprises a speaker or a piezo-type sound emitter and corresponding driving circuitry.

The piece of hygiene equipment 10-1 comprises further a code generation section 15 configured to generate a code as a reward token, that can be used as a voucher for making online purchases, free theatre or cinema tickets, free access to gyms and/or spas, and/or free consumption in a cafeteria and/or restaurant of, for example, the very premises at where the piece of hygiene equipment is installed. A code may be displayed as some sort of reward action for example for obtaining a free beverage at the hospital's cafeteria. The displayed code can be a human readable alphanumeric code or a one- or two-dimensional barcode (e.g. QR code) so that it can be read by, for example, a smartphone.

The generated code may generally represent a value token and should be generated so as to avoid unauthorized entities from generating or obtaining valid codes. This may be achieved by, for example, generating and optionally also encrypting codes ad hoc using a local key stored in the piece of hygiene equipment, obtaining codes from a list of pre-generated codes stored on the piece of hygiene equipment, or receiving codes from an external entity. Thus, on the display 31 a reward action is displayed as form of a code generated by the code generation section.

Figure 2B shows a further piece of hygiene equipment 10-2 which is provided with a closed compartment 33 (enclosure), which can accommodate a physical object 300 as a reward. Preferably, the enclosure 33 is equipped with a transparent door 34 and/or sidewall so that an operator may well see the reward object 300. Such objects may include gifts or giveaways, product samples, physical cinema or theatre tickets, physical vouchers, and the like. The reward action in this case will comprise an operation of opening the door 34 or, in more general terms, releasing the object 300 to an operator. This may include the driving of magnetic or electric actuators and/or motors.

Figure 2C shows a further example of a piece of hygiene equipment 10-3. The shown piece of hygiene equipment comprises a printer 35 which is arranged to print out a slip 36 which can be, in turn, a voucher, ticket, lottery ticket, or the like. In this context, the reward action will be the operation of the printer 35 which will be in turn the production of the output slip 36. The printed slip may display a code. The piece of hygiene equipment 10-3 comprises a code generation section as the device 10-1 of figure 2A, and the code is generated by a code generation section 15 (not shown) of the piece of hygiene equipment, as the reward action. The device 10-3 may further include an operator detection section 14 that is configured to determine a property of an operator using the hygiene equipment, and may comprise or be coupled to at least one of an operator detection sensor 14-1, and an input device 14-2.

For example, in this specific embodiment, the operator detection section 14 comprises a operator detection sensor 14-1 (e.g. near field communication receiver, Bluetooth™ receiver, RF-tag sensor, one- or two-dimensional barcode reader, etc.) shown on Figure 2C, and may detect from a token 41, a card or badge 42, or a mobile device 43 (e.g. a mobile phone, a tablet, a pager), etc. carried by the operator a property of the operator so as to be able to distinguish at least between two individuals, persons, or groups. The determined property of the operator may be information identifying an operator to be rewarded, or information identifying a group of persons to which the operator belongs. For example, in some situations one individual/group may be selected to be rewarded by a reward action, whilst another one is not. For example, hospital staff (to be rewarded) can be distinguished from visitors (not to be rewarded), and employees can be distinguished from management, etc. The control section may, in such situations, be further configured to suppress a reward action based on the determined property.

Thus, the property of the operator may be used to determine the control command, to control the reward action, to generate the code, or to forward the code toward the operator. Instead of, or in addition to the sensor, the operator detection section 14 may comprise an input device 14-2 and the operator may input information from which the operator property may be determined. The input device 14-2 may be any known input device such as a keypad, a keyboard, a touch screen etc. For example, the operator may input information identifying him- or her, such as an employee ID number, a name, a mobile telephone number, an e-mail address, etc. However, the sensor and/or input device may be external to the device 10-3, and the operator detection section 14 may be configured to obtain information from these to determine the property of the operator. Although shown in conjunction with Figure 2C, the above operator detection section and the corresponding configuration of the control section may be well combined with any other embodiment of the present invention.

Figure 2D shows a further piece of hygiene equipment 10-4 which comprises an operator detection section obtaining, as the property of the operator, identification information indicating an operator to be rewarded such as an employee ID number, a name, a telephone number, an e-mail address, identification information of a communication or social network (e.g. a device MAC address, a twitter handle, a URL of a profile etc.) or any other information identifying the operator, or a group of persons to which the operator belongs. For this purpose a communication section 16 may be employed that communicates with a device 43, 44 for retrieving the property of the operator. For example, a phone 43 may communicate any mentioned ID to the piece of hygiene equipment 10-4. Said communication section 16 may further be configured to forward a code toward the operator, based on the obtained operator identification information, so that the code may be used by the operator.

The code is generated by a code generation section in the piece of hygiene equipment and forwarded for example directly toward the operator by the communication section or it may be forwarded to an external device 44, such as a server which in turn stores the code and/or forwards it to the operator, or a machine delivering rewards upon use by the operator. For example, the piece of hygiene equipment may forward a code to a vending machine, and when the operator having the determined property uses the vending machine, a reward may be delivered.

Alternatively, the reward action may simply be a transmission of information to the server, and the server may generate a code instead. The piece of hygiene equipment or the server, as the case may be, may determine how to forward the code to the operator based on the determined property of the operator. For example, if the property of the operator is an email address, the server may cause an email including the code to be transmitted toward the determined email address. As another example, if a property of the operator is an indication that the operator belongs to a certain group of persons, the server may be configured to store the code on a database, in association with the group to which the operator belongs.

In any case, forwarding the code to an operator may encourage desired habits by the operator, when receiving the code or when using the code to obtain a reward. By generating the code and providing it directly to the operator (e.g. by displaying it, or printing it on a slip), the operator may be more comfortable using the piece of hygiene equipment, knowing that no information which may identify him or her would be shared with external entities. Similar effects may be achieved by embodiments forwarding the code directly to the operator.

Alternatively, forwarding the code to an external device such as a server storing codes in association with information identifying operators may be used to reinforce positive group reinforcement. The codes generated for a group of operators may be 'pooled' and used together to reward a whole group of operators, instead of, or in addition to individual rewards. For example, a shift of hospital workers may decide to group the codes they each receive in a pool.

Codes may allow monitoring of the compliance to hygiene rules by groups of people without causing individual operators to feel personally targeted, if the codes are not associated with individuals but with groups of operators. In addition, pools of codes may be used to create competition between groups of operators by forming contests between different groups, or by offering better rewards when the compliance level of a group is high, to further increase motivation to comply with hygiene rules.

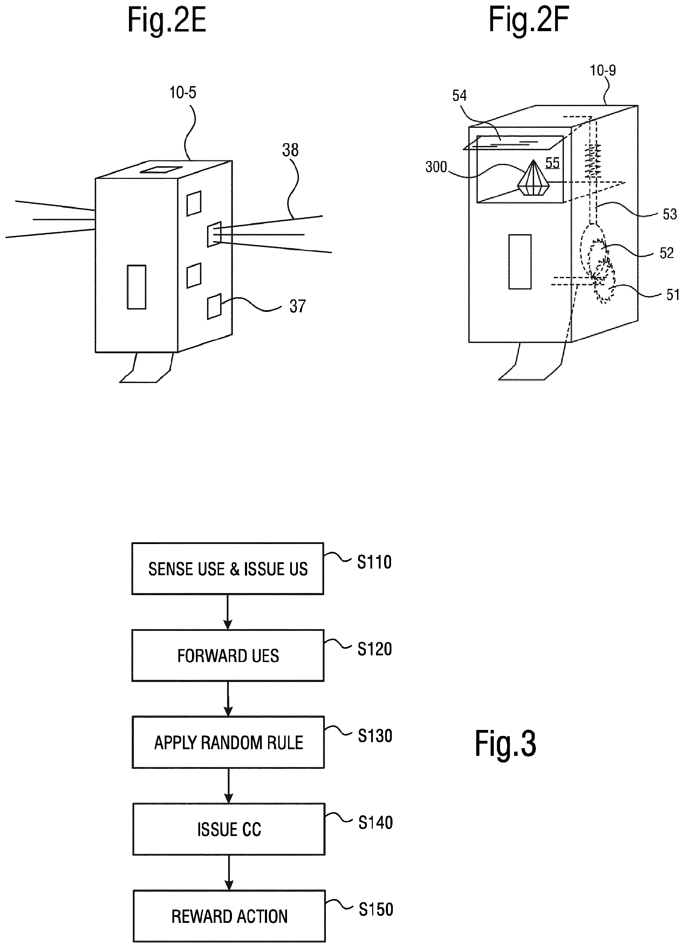

Figure 2E then shows a further piece of hygiene equipment 10-5 which is provided with light signal emitting elements 37 that are configured to emit various light signals 38 of various colors and flashing patterns. In this embodiment, the reward action will be the operation of the light-emitting elements 37 so as to emit a more or less predetermined light pattern comprising the light flashes 38. This example may be preferable in the sense that not only the individual operator using the equipment 10-5 becomes aware of the reward action but also individuals in the near vicinity can take note of the fact that a reward is taking place.

In general, however, it is noted that the embodiments of the present disclosure likewise envisage any suitable combination of circuits, mechanisms, and/or configurations that perform a reward action as they have been described in conjunction with figures 2A to 2E. The piece of hygiene equipment provides also a control section that is further configured to select a specific reward action generally from at least two alternative reward actions. In an embodiment this selection is made based on a day of time or a location of the rewarding hygiene equipment. For example, no sound signals or other annoying actions may be initiated at a nighttime (specific time period) or close to a patient bedside (specific location). However, if the time falls outside any specific time period, an alternative reward action may be selected that also comprises sound signals, printing a voucher, etc.

In the invention, there is provided a piece of hygiene equipment to be used by one or more operators, comprising a sensor section configured to generate a usage signal in response to an operator using the piece of hygiene equipment, a detection section configured to forward, in response to receiving said usage signal, a usage event signal to a determination section for determining a control command based on a random rule and said usage event signal, and a control section configured to control a reward action in response to receiving said control command.

The control section may comprise a processor that before controlling or initiating a reward action, awaits the detection of a usage event signal (UES) via an electrical connection/signal from the detection section. In a modification, the usage event signal (UES) is received as a usage signal (US) directly from the sensor section as a corresponding electrical signal. Upon receiving the usage event signal (UES) or the usage signal (US), the processor accesses signals from a time signal circuit or ADC circuit and determines at least some kind of value that represents a randomized, or at least quasi randomized, result in the sense that the value is impossible or at least difficult to predetermine. Based on this value the processor takes a decision whether a reward action is to be controlled, and, if so, the processor controls or initiates a reward action which includes the processor outputting electrical control signals toward corresponding hardware and circuitry for executing the reward action. For example, the electrical control signals may result in an audio, image, video, visible, and/or optical output, and/or in actuating mechanical actuators that, for example, open an enclosure.

In one embodiment, the control section may comprise a processor that before controlling or initiating a reward action, the processor accesses memory containing a database corresponding certain days of time to certain reward actions and a clock for determining the time of day; and then selects the reward action corresponding to the day of time. In another embodiment, the control section may comprise a processor that before controlling or initiating a reward action, the processor accesses memory containing a database corresponding certain locations to certain reward actions and a proximity sensor for determining the location of the hygiene product or its relative location to another object; and then selects the reward action corresponding to the certain location. In another embodiment, the control section may comprise a processor that, before controlling or initiating a reward action, accesses memory containing a database of certain inputs from an operator previously stored in the memory with certain reward actions. The processor may then select the reward action corresponding to the stored operator input.

When a code is generated, the determined property of the operator and the code may be associated with each other. As a result, the code may be used only by the operator or operators having the associated property, which may reduce the possibility of unwanted use of codes. The code may also be generated and/or encrypted in the piece of hygiene equipment and/or the server 44 based on the determined property. For example, the obtained property may be used as a public key to encrypt the code, and the operator may use a private key associated to the public key and known only to the operator, to decrypt the code and use it. The embodiments may use any public-key cryptography mechanism, to improve the encryption of the code.

In embodiments where the piece of hygiene equipment determines a property of the operator and a code is forwarded to the operator by the external device, as explained with reference to Figure 2D above, the reward action controlled by the control section of the piece of hygiene equipment may simply be a notification to the operator (or to individuals in the near vicinity) indicating that a code has been generated and forwarded to the operator.

In some embodiments a centralized server may receive codes forwarded by a plurality of pieces of hygiene equipment, or the centralized server may generate codes based on information transmitted by the plurality of pieces of hygiene equipment. In these embodiments, it is therefore possible to obtain codes or reward actions based on the usage of more than one piece of hygiene equipment.

In a way, the above described embodiments employ electric and electronical components for implementing at least in part some of sensing, detection, determination, and control actions. This may be in particular advantageous in view of the vast range of possibilities modern electronics offers at reasonable cost and effort (sound signals, blinking lights, display of text and/or images or videos, etc.). However, there are also embodiments of the present invention that take into account the necessity of at least one power supply (battery, solar cell, ultracap, energy harvesting device, connection to a power supply line, etc.) when electronics are involved.

In this context it is referred to Figure 2F that shows a schematic view of a piece of hygiene equipment according to a further embodiment of the invention that does not rely on electronics for providing a reward action, and, therefore may dispense with an electrical power supply. This embodiment, according to the invention, comprises a piece of hygiene equipment to be used by one or more operators, comprising means for determining a use of the piece of hygiene equipment by an operator, means for executing a control action based on a random rule in response to said use being determined, and means for executing a reward action in response to said control action.

Specifically, the shown piece of hygiene equipment 10-9 may comprise a mechanical arrangement 51 (levers, springs, gearwheels, and the like) that are arranged to determine a use of the piece of hygiene equipment by an operator. For example, the motion of a soap ejection lever can act on this mechanical arrangement 51. A further mechanism 52 can be configured to execute a control action based on a random rule in response to said use being determined, e.g. by receiving a force from arrangement 51. The random rule may in this case be implemented by a code wheel (wheel with randomly distributed notches or teeth) and/or a mechanic transmission that is arranged to distribute a relatively small number of reward events to a relatively large number of usage events in the sense of a decision pattern as mentioned also elsewhere in the present disclosure. Finally, the mechanism 52 may interact with, for example, a spring loaded lever 53 for executing a reward action in response to said control action in the form of opening a possibly also spring-loaded door 54 of an enclosure 55 to release access to a reward object 300. Again, the door 54 may be at least in part transparent so that the operators can see what kind of reward 300 is waiting. Naturally, also other mechanical reward actions can be executed, such as removing a cover from a code, playing a music box, and the like.

Figure 3 shows a flowchart of a general method embodiment of the present invention. This method is for operating hygiene equipment to be used by one or more operators and comprises a step S110 of generating a usage signal in response to an operator using the piece of hygiene equipment. Subsequently, in step S120, a usage event signal is forwarded to a determination section in response to receiving said usage signal. In step S130, a random rule is applied in response to receiving said usage event signal, and in step S140 it is determined whether or not a control command is to be issued. In step S150, a reward action is controlled in response to receiving said control command.

In a way, one or more embodiments of the present invention address a problem that there are many situations where a "management" would like to encourage others to clean their hands more often (e.g. hospital management wanting increased compliance from employees, teachers want students to wash before lunch...). The users/operators of the system tend to want to skip hygiene for reasons of time, convenience, sore hands etc. Existing solutions for encouraging hand hygiene may be either ineffective (e.g. posters) or labor intensive (education, behavior change, tracking and follow-up). In addition, many behavior and tracking systems result in feedback long after the hand hygiene occasion. Therefore, an embodiment of the present invention may provide a nudge toward proper behavior in a way which may require minimal effort and administration but which may be effective in achieving the goal and which may immediately be satisfying in order to encourage hand hygiene behavior.

In other words, an embodiment of the present invention ties a small reward to using a dispenser of hand hygiene products (soap, sanitizer, paper towels or lotion). In this way, the use of the dispenser is encouraged. In order to retain the impact of the reward, it should not be given every time the dispenser is used, and therefore a random rule is employed. The owner of the dispenser can choose how to dispense the reward (at a certain frequency, at a given time of day). In this way the owner of the dispenser can steer the timing of the reward to address possible issues or to pinpoint desired changes. It may be preferable that at least part of the reward is delivered at the moment of hand hygiene so that the desired behavior is reinforced.

In general, an embodiment may thus envisage a stand-alone solution (equipment with section 120) or the dispenser can be part of a larger system where the signal UES is forwarded to a remote determination of a reward. In all cases criteria for reward can be determined and set from a computer and can make use of existing compliance data. According to a further embodiment, the piece of equipment can respond to an individual id (i.e. badge or telephone) and reward each individual at a pre-determined frequency. In this case the reward can be delivered later (in this case there should also be an immediate reward such as blinking lights to generate the positive association to the behavior). In line with further embodiments of the present invention, the reward could be patterns of blinking lights, music, a physical object (small toy, movie ticket, etc.), a code to redeem a reward or to enter a contest. The code is displayed on a screen, and it can further be printed on an in-dispenser printer or sent by mail/text.

Although detailed embodiments have been described, these only serve to provide a better understanding of the invention defined by the independent claims and are not to be seen as The scope of the invention is indeed defined by the appended claims.

## Claims

1. A piece of hygiene equipment (10) to be used by one or more operators, comprising:
- a sensor section (11) configured to generate a usage signal (US) in response to an operator using the piece of hygiene equipment; and
- a detection section (12) configured to forward, in response to receiving said usage signal, a usage event signal (UES) to a determination section for determining a control command (CC) based on a random rule and said usage event signal;
- a control section (13) configured to control a reward action, in response to receiving said control command;
- a reward action execution section comprising a display (31); and
- a code generation section (15) configured to generate a code as a reward token,
wherein the reward action execution section is configured to display said code as the reward action on said display.

2. The piece of hygiene equipment according to claim 1, further comprising said determination section.

3. The piece of hygiene equipment according to claim 1, further comprising a communication section configured to forward said usage event signal to and receiving said control command from said determination section.

4. The piece of hygiene equipment according to any one of claims 1 to 3, wherein the reward action execution section further comprises any one of an optical signal section, an acoustic signal section, a sound generator, an audio output, a printer, and an enclosure adapted to accommodate an object.

5. The piece of hygiene equipment according to claim 4, comprising a printer and wherein the reward action execution section is configured to print said code on a slip by said printer as the reward action.

6. The piece of hygiene equipment according to any one of claims 1 to 5, further comprising an operator detection section (44) configured to determine a property (43) of an operator.

7. The piece of hygiene equipment according to claim 6, wherein the operator detection section is configured to obtain identification information indicating an operator to be rewarded, and the piece of hygiene equipment further comprises a communication section (16) configured to forward a code toward an operator based on said obtained operator identification information.

8. The piece of hygiene equipment according to any one of claims 1 to 7, wherein the determination section further applies a rule based on any one of a time span, a time, a usage frequency, a location of the piece of hygiene equipment.

9. The piece of hygiene equipment according to any one of claims 1 to 8, comprising an audio output and wherein the reward action execution section is configured to output a sound signal as the reward action on said audio output.

10. The piece of hygiene equipment according to any one of claims 1 to 9, comprising an enclosure and wherein the reward action execution section is configured to open a door to said enclosure as the reward action.

11. The piece of hygiene equipment according to claim 10, wherein said enclosure is at least in part transparent.

12. The piece of hygiene equipment according to any one of claims 1 to 11, wherein the control section is further configured to select a specific reward action.

13. The piece of hygiene equipment according to any one of claims 6 to 12, wherein the control section is further configured to suppress a reward action based on the determined property.

14. The piece of hygiene equipment according to any one of claims 1 to 13, wherein the piece of hygiene equipment is any one of a soap dispenser, a dispenser for disinfectant solutions, gels or substances, a towel dispenser, a glove dispenser, a tissue dispenser, a hand dryers, a sink, and a radiation assisted disinfectant point.

15. A method for operating hygiene equipment (10) to be used by one or more operators, comprising the steps of:
- generating (S110) a usage signal (US) in response to an operator using the piece of hygiene equipment;
- forwarding (S120), in response to receiving said usage signal, a usage event signal (UES) to a determination section (20) for determining (S140) a control command (CC) based on a random rule (S130) and said usage event signal;
- controlling a reward action (S150) in response to receiving said control command;
- generating a code as a reward token; and
- executing a reward action by displaying said code as the reward action on a display (31).

## Patentansprüche

1. Hygieneausrüstungsteil (10), das von einem oder mehreren Bedienern genutzt werden soll, umfassend:
- einen Sensorabschnitt (11), der dazu ausgebildet ist, in Reaktion darauf, dass ein Bediener das Hygieneausrüstungsteil nutzt, ein Nutzungssignal (NS) zu erzeugen; und
- einen Erfassungsabschnitt (12), der dazu ausgebildet ist, in Reaktion auf den Empfang des Nutzungssignals ein Nutzungsereignissignal (UES) an einen Bestimmungsabschnitt weiterzuleiten, um auf Basis einer Zufallsregel und des Nutzungsereignissignals einen Steuerbefehl (CC) zu bestimmen;
- einen Steuerungsabschnitt (13), der dazu ausgebildet ist, in Reaktion auf den Empfang des Steuerbefehls eine Belohnungsaktion zu steuern;
- einen Belohnungsaktions-Ausführungsabschnitt, der eine Anzeige (31) umfasst; und
- einen Codeerzeugungsabschnitt (15), der dazu ausgebildet ist, einen Code als eine Belohnungswertmarke zu erzeugen,
wobei der Belohnungsaktions-Ausführungsabschnitt dazu ausgebildet ist, den Code als die Belohnungsaktion auf der Anzeige anzuzeigen.

2. Hygieneausrüstungsteil nach Anspruch 1, weiter den Bestimmungsabschnitt umfassend.

3. Hygieneausrüstungsteil nach Anspruch 1, weiter einen Kommunikationsabschnitt umfassend, der dazu ausgebildet ist, das Nutzungsereignissignal an den Bestimmungsabschnitt weiterzuleiten, und von demselben den Steuerbefehl zu empfangen.

4. Hygieneausrüstungsteil nach einem der Ansprüche 1 bis 3, wobei der Belohnungsaktions-Ausführungsabschnitt weiter eines aus einem Abschnitt für optische Signale, einem Abschnitt für akustische Signale, einem Klangerzeuger, einem Audioausgang, einem Drucker und einem Gehäuse umfasst, das dazu geeignet ist, ein Objekt zu beherbergen.

5. Hygieneausrüstungsteil nach Anspruch 4, das einen Drucker umfasst, und wobei der Belohnungsaktions-Ausführungsabschnitt dazu ausgebildet ist, als die Belohnungsaktion den Code über den Drucker auf einen Zettel zu drucken.

6. Hygieneausrüstungsteil nach einem der Ansprüche 1 bis 5, weiter einen Bediener-Erfassungsabschnitt (44) umfassend, der dazu ausgebildet ist, eine Eigenschaft (43) eines Bedieners zu bestimmen.

7. Hygieneausrüstungsteil nach Anspruch 6, wobei der Bediener-Erfassungsabschnitt dazu ausgebildet ist, Identifikationsinformationen zu erhalten, die einen Bediener angeben, der belohnt werden soll, und das Hygieneausrüstungsteil weiter einen Kommunikationsabschnitt (16) umfasst, der dazu ausgebildet ist, auf Basis der erhaltenen Bediener-Identifikationsinformationen einen Code an einen Bediener weiterzuleiten.

8. Hygieneausrüstungsteil nach einem der Ansprüche 1 bis 7, wobei der Bestimmungsabschnitt weiter eine Regel anwendet, die auf einem aus einer Zeitspanne, einer Uhrzeit, einer Nutzungshäufigkeit, einem Standort des Hygieneausrüstungsteils basiert.

9. Hygieneausrüstungsteil nach einem der Ansprüche 1 bis 8, das einen Audioausgang umfasst, und wobei der Belohnungsaktions-Ausführungsabschnitt dazu ausgebildet ist, als die Belohnungsaktion ein Klangsignal am Audioausgang auszugeben.

10. Hygieneausrüstungsteil nach einem der Ansprüche 1 bis 9, das ein Gehäuse umfasst, und wobei der Belohnungsaktions-Ausführungsabschnitt dazu ausgebildet ist, als die Belohnungsaktion eine Klappe an dem Gehäuse zu öffnen.

11. Hygieneausrüstungsteil nach Anspruch 10, wobei das Gehäuse mindestens zum Teil durchsichtig ist.

12. Hygieneausrüstungsteil nach einem der Ansprüche 1 bis 11, wobei der Steuerungsabschnitt weiter dazu ausgebildet ist, eine spezifische Belohnungsaktion auszuwählen.

13. Hygieneausrüstungsteil nach einem der Ansprüche 6 bis 12, wobei der Steuerungsabschnitt weiter dazu ausgebildet ist, auf Basis der bestimmten Eigenschaft eine Belohnungsaktion zu unterdrücken.

14. Hygieneausrüstungsteil nach einem der Ansprüche 1 bis 13, wobei es sich bei dem Hygieneausrüstungsteil um eines aus einem Seifenspender, einem Spender für Desinfektionsmittellösungen, -gele oder -substanzen, einem Handtuchspender, einem Handschuhspender, einem Papierspender, einem Handtrockner, einem Waschbecken und einer strahlungsunterstützten Desinfektionsstelle handelt.

15. Verfahren für den Betrieb von Hygieneausrüstung (10), die von einem oder mehreren Bedienern genutzt werden soll, umfassend die Schritte des:
- Erzeugens (S110) eines Nutzungssignals (US) in Reaktion darauf, dass ein Bediener das Hygieneausrüstungsteil nutzt;
- Weiterleitens (S120) eines Nutzungsereignissignals (UES) in Reaktion auf den Empfang des Nutzungssignals an einen Bestimmungsabschnitt (20), um auf Basis einer Zufallsregel (S130) und des Nutzungsereignissignals einen Steuerbefehl (CC) zu bestimmen (S140);
- Steuerns einer Belohnungsaktion (S150) in Reaktion auf den Empfang des Steuerbefehls;
- Erzeugens eines Codes als eine Belohnungswertmarke; und
- Ausführens einer Belohnungsaktion durch Anzeigen des Codes als die Belohnungsaktion auf einer Anzeige (31).

## Revendications

1. Article d'équipement d'hygiène (10) devant être utilisé par un ou plusieurs opérateurs, comprenant :
- une section à capteur (11) configurée pour générer un signal d'utilisation (US) en réponse à un opérateur utilisant l'article d'équipement d'hygiène ; et
- une section de détection (12) configurée pour envoyer, en réponse à la réception dudit signal d'utilisation, un signal d'événement d'utilisation (UES) à une section de détermination permettant de déterminer une instruction de commande (CC) sur la base d'une règle aléatoire et dudit signal d'événement d'utilisation ;
- une section de commande (13) configurée pour commander une action de récompense, en réponse à la réception de ladite instruction de commande ;
- une section d'exécution d'action de récompense comprenant un écran (31) ; et
- une section de génération de code (15) configurée pour générer un code en tant que jeton de récompense,
dans lequel la section d'exécution d'action de récompense est configurée pour afficher ledit code en tant qu'action de récompense sur ledit écran.

2. Article d'équipement d'hygiène selon la revendication 1, comprenant en outre ladite section de détermination.

3. Article d'équipement d'hygiène selon la revendication 1, comprenant en outre une section de communication configurée pour envoyer ledit signal d'événement d'utilisation à et recevant ladite instruction de commande à partir de ladite section de détermination.

4. Article d'équipement d'hygiène selon l'une quelconque des revendications 1 à 3, dans lequel la section d'exécution d'action de récompense comprend en outre l'un quelconque d'une section de signal optique, une section de signal acoustique, un générateur de son, une sortie audio, une imprimante et une enceinte adaptée pour contenir un objet.

5. Article d'équipement d'hygiène selon la revendication 4, comprenant une imprimante et dans lequel la section d'exécution d'action de récompense est configurée pour imprimer ledit code sur un morceau de papier par ladite imprimante en tant qu'action de récompense.

6. Article d'équipement d'hygiène selon l'une quelconque des revendications 1 à 5, comprenant en outre une section de détection d'opérateur (44) configurée pour déterminer une propriété (43) d'un opérateur.

7. Article d'équipement d'hygiène selon la revendication 6, dans lequel la section de détection d'opérateur est configurée pour obtenir des informations d'identification indiquant un opérateur devant être récompensé, et l'article d'équipement d'hygiène comprend en outre une section de communication (16) configurée pour envoyer un code vers un opérateur sur la base desdites informations d'identification d'opérateur obtenues.

8. Article d'équipement d'hygiène selon l'une quelconque des revendications 1 à 7, dans lequel la section de détermination applique en outre une règle sur la base de l'un quelconque d'une période de temps, d'une heure, d'une fréquence d'utilisation, d'un emplacement de l'article d'équipement d'hygiène.

9. Article d'équipement d'hygiène selon l'une quelconque des revendications 1 à 8, comprenant une sortie audio et dans lequel la section d'exécution d'action de récompense est configurée pour sortir un signal sonore en tant qu'action de récompense sur ladite sortie audio.

10. Article d'équipement d'hygiène selon l'une quelconque des revendications 1 à 9, comprenant une enceinte et dans lequel la section d'exécution d'action de récompense est configurée pour ouvrir une porte vers ladite enceinte en tant qu'action de récompense.

11. Article d'équipement d'hygiène selon la revendication 10, dans lequel ladite enceinte est au moins en partie transparente.

12. Article d'équipement d'hygiène selon l'une quelconque des revendications 1 à 11, dans lequel la section de commande est en outre configurée pour sélectionner une action de récompense spécifique.

13. Article d'équipement d'hygiène selon l'une quelconque des revendications 6 à 12, dans lequel la section de commande est en outre configurée pour supprimer une action de récompense sur la base de la propriété déterminée.

14. Article d'équipement d'hygiène selon l'une quelconque des revendications 1 à 13, dans lequel l'article d'équipement d'hygiène est l'un quelconque d'un distributeur de savon, d'un distributeur pour des solutions, gels ou substances désinfectants, d'un distributeur de serviettes, d'un distributeur de gants, d'un distributeur de mouchoirs en papier, de sèche-mains, d'un lavabo et d'un point de désinfection par rayonnement.

15. Procédé de fonctionnement d'un équipement d'hygiène (10) devant être utilisé par un ou plusieurs opérateurs, comprenant les étapes consistant à :
- générer (S110) un signal d'utilisation (US) en réponse à un opérateur utilisant l'article d'équipement d'hygiène ;
- envoyer (S120), en réponse à la réception dudit signal d'utilisation, un signal d'événement d'utilisation (UES) à une section de détermination (20) permettant de déterminer (S140) une instruction de commande (CC) sur la base d'une règle aléatoire (S130) et dudit signal d'événement d'utilisation ;
- commander une action de récompense (S150) en réponse à la réception de ladite instruction de commande ;
- générer un code en tant que jeton de récompense ; et
- exécuter une action de récompense en affichant ledit code en tant qu'action de récompense sur un écran (31).
